# FASCICULE DE BREVET EUROPEEN

(11) **EP 3 752 484 B1**
(45) Date de publication et mention de la délivrance du brevet: **24.11.2021**
(21) Numéro de dépôt: 19717342.0
(22) Date de dépôt: 20.02.2019
(51) Int. Cl.: C07C 303/06, C07C 309/04

(54) **PROCÉDÉ DE SYNTHÈSE INDUSTRIELLE EN CONTINU D'ACIDE ALCANE-SULFONIQUE**
INDUSTRIELLES VERFAHREN ZUR KONTINUIERLICHEN SYNTHESE VON ALKANSULFONSÄURE
INDUSTRIAL PROCESS FOR CONTINUOUSLY SYNTHESIZING ALKANE-SULPHONIC ACID

(30) Priorité: 14.02.2018 FR 1851230
(43) Date de publication de la demande: 23.12.2020
(73) Titulaire: ARKEMA FRANCE, 92700 Colombes (FR)
(72) Inventeur: DUBOIS, Jean-Luc, 92700 COLOMBES (FR)
(74) Mandataire: Arkema Patent
(86) Numéro de dépôt international: PCT/FR2019/050393
(87) Numéro de publication internationale: WO 2019/158892

(56) Documents cités:
- WO-A1-2005/044789
- WO-A2-2005/069751
- SUDIP MUKHOPADHYAY ET AL: "A High-Yield, Liquid-Phase Approach for the Partial Oxidation of Methane to Methanol using SO3 as the Oxidant", ADVANCED SYNTHESIS & CATALYSIS, vol. 347, no. 9, 1 juillet 2005 (2005-07-01), pages 1203-1206, XP055110191, ISSN: 1615-4150, DOI: 10.1002/adsc.200404394

## Description

La présente invention concerne le domaine de la thiochimie, plus précisément de la synthèse industrielle de dérivés soufrés, en particulier d'acides sulfoniques, et notamment d'acides alcane-sulfoniques. Plus particulièrement, la présente invention concerne un procédé de préparation industrielle d'acide alcane-sulfonique à partir d'alcanes.

Les procédés de synthèse industrielle de produits chimiques sont constamment améliorés, afin d'en réduire les risques pour l'environnement, les consommations énergétiques, les sous-produits, les déchets produits, et aussi augmenter les rendements, les degrés de pureté des produits obtenus, et autres.

Les procédés industriels pour la préparation des produits soufrés n'échappent pas à cette règle. La chimie du soufre, encore appelée thiochimie, est tout particulièrement visée, en raison des caractères souvent toxiques et odorants des produits de synthèse, des intermédiaires, ainsi qu'éventuellement des sous-produits formés.

Les consommations toujours grandissantes de produits chimiques dans le monde imposent également des capacités de production de plus en plus importantes. Afin d'augmenter les capacités de productions, on peut envisager une « augmentation physique » (construction de nouvelles unités de production ou agrandissement d'unités de production existantes) et/ou une « augmentation chimique », c'est-à-dire une modification substantielle des procédés existants et actuellement utilisés, afin d'améliorer les rendements de production, les sélectivités, et autres, comme rappelé ci-dessus.

La modification ou le remplacement de procédés industriels existants et utilisés ne va pas cependant sans poser de nombreux problèmes et les industriels imposent des tests très poussés en laboratoire puis à l'échelle d'un pilote industriel pour s'assurer de la viabilité dudit nouveau procédé à l'échelle industrielle.

Les acides alcane-sulfoniques, et en particulier l'acide méthane-sulfonique de formule CH₃-SO₃H, sont aujourd'hui préparés sur le plan industriel selon des procédés bien connus et datant pour la plupart de plusieurs décennies, avec des rendements qui pourraient avantageusement être améliorés, sans parler des sous-produits non désirés qui doivent être traités ou valorisés, avec des coûts associés non compatibles avec les conditions économiques actuelles.

Malgré tous les efforts déployés les dernières décennies, les procédés industriels permettant l'accès aux acides alcane-sulfoniques et à l'acide méthane-sulfonique en particulier, souffrent pour certains de nombreux inconvénients, et/ou de rendements qui pourraient encore être améliorés.

Il reste par conséquent un besoin pour un procédé industriel simple, efficace, économique et rentable sur le plan industriel pour la préparation d'acide alcane-sulfonique et d'acide méthane-sulfonique en particulier.

Les demandes de brevets US2006100458 et US2005070614 décrivent la préparation d'acide méthane-sulfonique (CH₃SO₃H) à partir de méthane (CH4) et de trioxyde de soufre (SO₃). Les réactions décrites dans ces deux documents sont cependant toutes réalisées en mode batch dans des autoclaves, souvent de petites tailles, sous atmosphère inerte, par exemple sous azote. Les autoclaves sont chauffées, par exemple jusqu'à environ 80°C, avec des pressions en méthanecomprises entre 2 MPa (300 psi) et près de 10 MPa (1400 psi). Il est évident que de telles conditions réactionnelles, en présence de tels réactifs de synthèse, rendent ledit procédé de préparation d'acide méthane-sulfonique à partir de méthane et de trioxyde de soufre, peu envisageable sur le plan industriel.

Il pourrait cependant être très intéressant de pouvoir convertir le méthane, un gaz naturel, en acide méthane-sulfonique (AMS) en présence de trioxyde de soufre (SO₃).

Il a maintenant été trouvé qu'il est possible de convertir un alcane en acide alcane-sulfonique correspondant, par exemple le méthane en acide méthane-sulfonique, en présence de trioxyde de soufre, dans un procédé continu, de manière simple, efficace, avec de bons rendements. En outre le procédé de l'invention peut très facilement être opéré à l'échelle industrielle. Le procédé de l'invention permet également d'atteindre des degrés de pureté en acide alcane-sulfonique supérieurs à 95%, voire supérieurs à 98%.

D'autres avantages encore liés à la présente invention apparaîtront à la lumière de la description qui suit.

Ainsi et selon un premier aspect, la présente invention concerne un procédé de préparation industriel en continu d'un acide alcane-sulfonique, à partir de l'alcane correspondant et de trioxyde de soufre, ledit procédé étant réalisé dans un solvant qui est ledit acide alcane sulfonique.

Par « alcane correspondant », on entend l'alcane comportant le même nombre d'atomes de carbone que l'acide alcane-sulfonique que l'on souhaite fabriquer en continu sur le plan industriel. À titre d'exemples non limitatifs, le méthane (CH4) est utilisé pour préparer l'acide méthane sulfonique (CH₃SO₃H), l'éthane (C₂H₆) est utilisé pour préparer l'acide éthane-sulfonique (C₂H₅SO₃H), le propane (C₃H₈) est utilisé pour préparer l'acide propane-sulfonique (C₃H₇SO₃H), le butane (C₄H₁₀) est utilisé pour préparer l'acide butane-sulfonique (C₃H₇SO₃H), et ainsi de suite, et plus généralement l'alcane de formule CₙH₂ₙ₊₂ est utilisé pour préparer l'acide alcane sulfonique de formule (CₙH₂ₙ₊₁SO₃H).

La présente invention concerne ainsi un procédé de préparation industriel en continu d'un acide alcane sulfonique de formule (CₙH₂ₙ₊₁SO₃H), dans laquelle « n » représente un entier compris entre 1 et 20 bornes incluses, de préférence entre 1 et 12 bornes incluses, de préférence encore entre 1 et 6 bornes incluses, et par exemple égal à 1, 2, ou 3.

Le procédé selon la présente invention est tout particulièrement adapté pour la synthèse industrielle en continu d'acide méthane-sulfonique à partir de méthane et de trioxyde de soufre.

Le procédé selon la présente invention se caractérise par le fait qu'il est réalisé en milieu acide alcane-sulfonique que l'on souhaite préparer en continu.

Plus spécifiquement, la présente invention concerne le procédé de préparation industriel en continu d'acide alcane-sulfonique de formule CₙH2ₙ₊₁SO₃H, à partir de l'alcane de formule CₙH₂ₙ₊₂, où n est tel que défini précédemment, ledit procédé comprenant au moins les étapes suivantes :
a) chargement d'acide alcane-sulfonique CₙH2ₙ₊₁SO₃H, où n est tel que défini précédemment, dans un réacteur,
b) chauffage dudit acide alcane-sulfonique à une température comprise entre 25°C et 120°C, de préférence comprise entre 25°C et 100° Cde préférence encore comprise entre 25°C et 70°C,
c) introduction d'une quantité de trioxyde de soufre de sorte que le ratio molaire (acide alcane-sulfonique)/(trioxyde de soufre) soit compris entre 70/30 et 95/5,
d) introduction de l'alcane sous une pression comprise entre 4 MPa et 15 MPa, de préférence comprise entre 5 MPa et 12 MPa,
d) introduction d'un initiateur radicalaire,
e) conduite de la réaction en continu, en opérant de manière simultanée, séquencée ou alternative les trois sous-étapes e1), e2) et e3) suivantes :
   e1) ajout de trioxyde de soufre de manière à maintenir le ratio molaire (acide alcane-sulfonique)/(trioxyde de soufre) initial défini à l'étape c),
   e2) ajout de l'alcane de manière à maintenir un ratio molaire (alcane)/(trioxyde de soufre) compris entre 4/1 et 2/1, de préférence entre 3,5/1 et 2,5/1, typiquement de manière à maintenir un ratio molaire (alcane)/(trioxyde de soufre) égal à environ 3, et
   e3) soutirage en continu de l'acide alcane-sulfonique de formule CₙH₂ₙ₊₁SO₃H, et f) récupération de l'acide alcane-sulfonique de formule CₙH₂ₙ₊₁SO₃H après séparation éventuelle des réactifs n'ayant pas réagi, et des éventuels sous-produits non désirés.

La température réactionnelle est généralement comprise entre 25°C et 120°C, de préférence comprise entre 25°C et 100°C, de préférence encore comprise entre 25°C et 70°C, typiquement entre 30°C et 60°C, par exemple eviron 50°C. La température réactionnelle peut bien entendu être aisément adaptée par l'homme du métier en fonction du type d'alcane mis en œuvre et/ou de la nature de l'acide alcane-sulfonique à préparer, mais aussi du type de réacteur utilisé et de la pression réactionnelle.

La pression réactionnelle est généralement comprise entre 4 MPa et 15 MPa, de préférence comprise entre 5 MPa et 12 MPa, et peut aisément être adaptée par l'homme du métier en fonction du type d'alcane mis en œuvre et/ou de la nature de l'acide alcane-sulfonique à préparer, mais aussi du type de réacteur utilisé et de la température réactionnelle. Il est préférable d'opérer avec une pression d'alcane de manière à favoriser la solubilisation dudit alcane dans l'acide alcane-sulfonique qui sert de solvant. On opère par exemple avec une pression voisine de 10 MPa lorsque l'alcane est le méthane est le solvant de la réaction est l'acide méthane-sulfonique.

Le procédé industriel en continu de la présente invention peut être réalisé dans tout type de réacteur bien connu de l'homme du métier et adapté pour ce type de réaction, les réactifs de départ et le produit obtenu. Parmi les modèles de réacteurs tout à fait appropriés et adaptés pour le procédé de la présente invention, on peut citer, de manière non limitative les réacteurs munis d'une boucle de recirculation, souvent dénommée « tourne-en-rond » (« loop reactors » en langue anglaise), les réacteurs à turbine auto-aspirante, les réacteurs munis d'un système dit à venturi, les réacteurs à lit rotatif (« spinning bed reactors » en langue anglaise), les réacteurs tubulaires, et autres.

Il est possible d'utiliser des réacteurs comportant un ou plusieurs des systèmes indiqués ci-dessus. Les réacteurs munis d'une boucle de recirculation sont tout particulièrement bien adaptés pour le procédé industriel en continu selon l'invention, et notamment ceux commercialisés par la société Buss, comme indiqué par exemple sur le site internet de ladite société Buss : http://www.buss-ct.com/buss_loop_reactor.html).

L'initiateur radicalaire utilisé dans le procédé de la présente invention peut également être de tout type connu de l'homme du métier et par exemple choisi parmi, de manière non limitative, l'azo-*bis*-*iso*-butyronitrile, les peroxydes minéraux ou organiques, tels que le peroxyde d'hydrogène (H₂O₂), le sulfate de mercure (HgSO₄), le sulfate de palladium (PdSO₄), le sulfate de césium (Ce(SO₄)₂), le K₂P₂O₈, le CaO₂, les halogènes tels que Br2, Cl₂, et I₂, en présence de chlorures métalliques tels que le chlorure de calcium, le chlorure de fer, et tout particulièrement le trichlorure de rhodium (RhCl₃), et également ceux décrits dans les demandes EP1558353, WO2015071351, WO2015071365, WO2015071371, et WO2015071455, et tout particulièrement les peroxydes de bis-(alcane-sulfonyle) de formule CₙH₂ₙ₊₁-SO₂-O-O-SO₂O-CₙH₂ₙ₊₁ (où n est tel que défini précédemment), les initiateurs de formule CₙH₂ₙ₊₁-SO₂-O-O-SO₂OX (où n est tel que défini précédemment et X représente l'hydrogène, un métal alcalin ou alcalino-terreux, le zinc ou l'aluminium), l'acide de Caro (HO-SO₃-OH) et ses sels avec un ou plusieurs métaux alcalin ou alcalino-terreux, et l'acide de Marshall (HO-SO₃-SO₃-OH) et ses sels avec un ou plusieurs métaux alcalin ou alcalino-terreux, par exemple son di-sel de potassium K⁺,⁻O-SO₃-SO₃-O⁻,K⁺.

Certains initiateurs, comme par exemple l'acide de Marshall, peuvent être générés *in situ.* Dans le cas de l'acide de Marshall, il peut être généré *in situ* par réaction de trioxyde de soufre avec le peroxyde d'hydrogène.

On préfère tout particulièrement utiliser les initiateurs de formule CₙH₂ₙ₊₁-SO₂-O-O-SO₂O-CₙH₂ₙ₊₁ (où n est tel que défini précédemment), les initiateurs de formule CₙH₂ₙ₊₁-SO₂-O-O-SO₂OX (où n est tel que défini précédemment et X représente l'hydrogène, un métal alcalin ou alcalino-terreux, le zinc ou l'aluminium), l'acide de Caro (HO-SO₃-OH) et ses sels avec un ou plusieurs métaux alcalin ou alcalino-terreux, et l'acide de Marshall (HO-SO3-SO3-OH) et ses sels avec un ou plusieurs métaux alcalin ou alcalino-terreux, par exemple son di-sel de potassium K⁺,⁻O-SO₃-SO₃-O⁻,K⁺.

Il peut être avantageux de dissoudre les initiateurs qui se présentent sous forme solide, avant de les introduire dans le milieu réactionnel. La dissolution peut s'effectuer dans tout solvant compatible avec ledit milieu réactionnel, par exemple dans un solvant protique, qui peut être le solvant de la réaction lui-même ou bien l'eau.

L'alcane de formule CₙH₂ₙ₊₂, où n est tel que défini précédemment, est alors ajouté de sorte que la réaction avec le trioxyde de soufre dans le solvant acide alcane-sulfonique peut commencer. Il doit être compris que l'initiateur peut être ajouté avant et/ou après et/ou encore pendant la première introduction de l'alcane.

L'alcane peut être introduit sous forme liquide ou gazeuse. Par exemple, lorsque l'alcane est le méthane, celui-ci peut être introduit par bullage dans la solution de trioxyde de soufre dans l'acide méthane-sulfonique.

Le réacteur utile pour le procédé de la présente invention peut être muni de tout système d'agitation bien connu de l'homme du métier, et par exemple, et à titre d'exemples non limitatifs, un ou plusieurs systèmes choisis parmi les agitateurs à hélices, les pompes de circulation, et dans le cas de réacteurs tubulaires, les chicanes, les systèmes oscillatoires, et autres.

Il doit également être compris que la quantité d'acide alcane-sulfonique formée dépend de la quantité d'alcane introduite, tout en s'assurant de maintenir le ratio molaire (alcane de formule CₙH₂ₙ₊₂)/(trioxyde de soufre) compris entre 4/1 et 2/1, de préférence entre 3,5/1 et 2,5/1, typiquement environ égal à 3, et de maintenir le ratio molaire (acide alcane-sulfonique)/(trioxyde de soufre) entre 70/30 et 95/5.

La conduite du procédé selon l'invention en continu est donc réalisée par ajout d'alcane et de trioxyde de soufre au fur et à mesure de leur consommation et de la quantité d'acide alcane-sulfonique soutiré, en maintenant le ratio molaire (alcane de formule CₙH₂ₙ₊₂)/(trioxyde de soufre) compris entre 4/1 et 2/1, de préférence entre 3,5/1 et 2,5/1, typiquement environ égal à 3, et le ratio molaire (acide alcane-sulfonique)/(trioxyde de soufre) entre 70/30 et 95/5, comme indiqué précédemment.

Le réacteur de la présente invention peut donc avantageusement comprendre plusieurs analyseurs en ligne permettant de suivre en continu les différentes teneurs en espèces présentes dans le système réactionnel afin d'asservir si nécessaire les vannes d'admission et de soutirage nécessaires pour la bonne conduite de la réaction selon les paramètres définis ci-dessus.

Le procédé selon la présente invention présente de nombreux avantages parmi lesquels on peut citer, outre le fait qu'il est réalisé de manière continue, une bonne sélectivité, et donc la génération d'une faible quantité de sous-produits indésirables, voire pas de sous-produits indésirables.

Le procédé de la présente invention est de préférence opéré dans des conditions substantiellement anhydres, et on préfère utiliser un solvant réactionnel (l'acide alcane-sulfonique) contenant la plus faible quantité d'eau possible, voire une acide alcane-sulfonique anhydre. De même, on préfère du trioxyde de soufre sous forme anhydre, l'utilisation d'oléum, qui est un mélange de trioxyde de soufre et d'acide sulfurique, n'est pas préférée pour la conduite du procédé de la présente invention.

Sans vouloir être lié par la théorie, la présence d'eau peut générer de l'acide sulfurique, sous-produit qui peut être non désirable, auquel cas il est nécessaire de prévoir une étape de séparation, par distillation par exemple, ce qui entraîne des coûts de production supplémentaires. La présence de traces d'eau, notamment provenant de l'initiateur radicalaire, peut cependant être acceptable dans la mesure où l'acide sulfurique sous-produit en quantité stœchiométrique par rapport à l'eau présente, est soutiré en continu avec l'acide alcane-sulfonique formé. Dès lors que la totalité de l'eau présente a été consommée, le sous-produit acide sulfurique ne sera plus formé.

Ainsi le procédé de la présente invention peut fonctionner de manière tout à fait aisée en continu, en ajoutant en continu l'alcane et le trioxyde de soufre et en soutirant en continu l'acide alcane-sulfonique formé.

Un autre avantage du procédé de la présente invention réside dans le fait que le trioxyde de soufre est présent dans le milieu réactionnel en très faible quantité, et notamment en quantité très inférieure à celle constatée dans la demande de brevet EP1558353. En effet, et sans vouloir être lié par la théorie, la cinétique de réaction de l'alcane avec le trioxyde de soufre est relativement lente, et il apparaît avantageux de réaliser la réaction de l'alcane avec le trioxyde de soufre dans un milieu dilué, ceci permettant d'augmenter la sélectivité et donc d'éviter la formation de sous-produits non désirables.

L'acide alcane-sulfonique soutiré en sortie de réacteur peut contenir des quantités plus ou moins importantes de produits de départ (alcane et trioxyde de soufre) n'ayant pas réagi. Ceux-ci peuvent être facilement séparés de l'acide alcane-sulfonique formé par stripping, par exemple par stripping thermique, et ainsi très avantageusement être facilement ré-injectés dans le milieu réactionnel.

L'acide alcane-sulfonique ainsi produit selon le procédé de la présente invention présente une grande pureté, supérieure à 95%, avantageusement supérieure à 98%, et même supérieure à 99%.

En particulier l'acide alcane-sulfonique formé est exempt de sous-produit est peut-être utilisé tel que, sans qu'il soit nécessaire de le purifier, par les techniques classiquement utilisées, notamment la distillation et/ou la recristallisation. Par « exempt », on entend teneur en impuretés inférieures à 1% en poids.

Le procédé de la présente invention est tout particulièrement adapté pour la synthèse industrielle en continu d'acide méthane-sulfonique à partir de méthane et de trioxyde de soufre, en milieu solvant méthane-sulfonique. Ainsi, et selon un mode de réalisation tout particulièrement préféré, le solvant est l'acide méthane-sulfonique, l'alcane est le méthane et le produit soutiré en continu est l'acide méthane-sulfonique.

Les acides alcane-sulfoniques trouvent des utilisations dans de très nombreux domaines d'application, telles que par exemple, et pour ne citer que quelques applications de l'acide méthane-sulfonique, le nettoyage, la catalyse, la synthèse en tant que réactif pour la préparation de molécules ou intermédiaires de synthèse de molécules, et autres.

Concernant plus spécifiquement l'acide méthane-sulfonique, celui-ci peut donc avantageusement être utilisé tel quel ou encore soumis à une étape de thermolyse à une température d'environ 200°C à 400°C, comme indiquépar exemple dans la demande de brevet EP1558353, afin de former du méthanol d'une part et du dioxyde de soufre d'autre part, le dioxyde de soufre pouvant très avantageusement être régénéré en trioxyde de soufre qui peut être ré-introduit dans le réacteur de synthèse du procédé de la présente invention.

L'invention concerne également le procédé de synthèse de méthanol comprenant les étapes a) à f) précédemment décrites où le solvant est l'acide méthane-sulfonique et l'alcane est le méthane, et une étape g) de chauffage pour former du dioxyde de soufre d'une part et du méthanol d'autre part.

L'invention a enfin pour objet un milieu réactionnel comprenant un acide alcane-sulfonique de formule CₙH₂ₙ₊₁SO₃H, où n est un entier compris entre 1 et 20 bornes incluses, de préférence entre 1 et 12 bornes incluses, de préférence encore entre 1 et 6 bornes incluses, et par exemple égal à 1, 2 ou 3, du trioxyde de soufre SO₃, et un alcane de formule CₙH₂ₙ₊₂, où n est tel que défini précédemment, milieu réactionnel dans lequel :
- le ratio molaire (acide alcane-sulfonique)/(SO₃) est compris entre 70/30 et 95/5, et
- le ratio molaire (alcane)/(SO₃) est compris entre 4/1 et 2/1, de préférence entre 3,5/1 et 2,5/1, typiquement égal à environ 3.

De manière tout particulièrement préféré, la présente invention concerne un milieu réactionnel comprenant de l'acide méthane-sulfonique (CH₃SO₃H), du trioxyde de soufre SO₃, et du méthane (CH4) dans lequel :
- le ratio molaire (acide méthane-sulfonique)/(trioxyde de soufre) est compris entre 70/30 et 95/5, et
- le ratio molaire (méthane)/(trioxyde de soufre) est compris entre 4/1 et 2/1, de préférence entre 3,5/1 et 2,5/1, typiquement égal à environ 3.

Le milieu réactionnel précité comprenant un acide alcane-sulfonique, du trioxyde de soufre SO₃, et un alcane, peut également comprendre d'autres espèces parmi lesquelles on peut citer notamment l'eau, l'acide sulfurique, l'initiateur radicalaire et/ou ses produits de décomposition. Ces autres espèces, lorsqu'elles sont présentes, sont le plus souvent sous forme de traces, et plus précisément leurs teneurs respectives n'excèdent pas 1 % en poids par rapport au poids total du milieu réactionnel, de préférence n'excèdent pas 1000 ppm poids par rapport au poids total du milieu réactionnel.

### Exemple :

Le procédé suivant est mis en œuvre :
a) chargement d'acide méthane sulfonique (AMS), dans un réacteur continu sous pression agité en fonctionnement stabilisé,
b) chauffage de l'acide méthane sulfonique à une température d'environ 50°C,
c) introduction d'une quantité de trioxyde de soufre de sorte que le ratio molaire (AMS)/(trioxyde de soufre) soit de 80/20,
d) introduction de méthane sous pression,
d) introduction en continu de l'initiateur CH₃-SO₂OO-SO₂OH,
e) conduite de la réaction en continu, en opérant de manière simultanée, séquencée ou alternative les trois sous-étapes e1), e2) et e3) suivantes :
   e1) ajout de trioxyde de soufre de manière à maintenir le ratio molaire (AMS)/(trioxyde de soufre) de 80/20,
   e2) ajout de méthane de manière à maintenir un ratio molaire (méthane)/(trioxyde de soufre) d'environ 3, et
   e3) soutirage en continu de l'acide méthane sulfonique.

## Revendications

1. Procédé de préparation industriel en continu d'acide alcane-sulfonique de formule CₙH₂ₙ₊₁SO₃H, à partir de l'alcane de formule CₙH₂ₙ₊₂, où n représente un entier compris entre 1 et 20 bornes incluses, de préférence entre 1 et 12 bornes incluses, de préférence encore entre 1 et 6 bornes incluses, et par exemple égal à 1, 2 ou 3, ledit procédé comprenant au moins les étapes suivantes :
a) chargement d'acide alcane-sulfonique CₙH₂ₙ₊₁SO₃H, où n est tel que défini précédemment, dans un réacteur,
b) chauffage dudit acide alcane-sulfonique à une température comprise entre 25°C et 120°C, de préférence comprise entre 25°C et 100° C, de préférence encore comprise entre 25°C et 70°C,
c) introduction d'une quantité de trioxyde de soufre de sorte que le ratio molaire (acide alcane-sulfonique)/(trioxyde de soufre) soit compris entre 70/30 et 95/5,
d) introduction de l'alcane sous une pression comprise entre 4 MPa et 15 MPa, de préférence comprise entre 5 MPa et 12 MPa,
d) introduction d'un initiateur radicalaire,
e) conduite de la réaction en continu, en opérant de manière simultanée, séquencée ou alternative les trois sous-étapes e1), e2) et e3) suivantes :
e1) ajout de trioxyde de soufre de manière à maintenir le ratio molaire (acide alcane-sulfonique)/(trioxyde de soufre) initial défini à l'étape c),
e2) ajout de l'alcane de manière à maintenir un ratio molaire (alcane)/(trioxyde de soufre) compris entre 4/1 et 2/1, de préférence entre 3,5/1 et 2,5/1, typiquement de manière à maintenir un ratio molaire (alcane)/(trioxyde de soufre) égal à environ 3, et
e3) soutirage en continu de l'acide alcane-sulfonique de formule CₙH₂ₙ₊₁SO₃H, et
f) récupération de l'acide alcane-sulfonique de formule CₙH₂ₙ₊₁SO₃H après séparation éventuelle des réactifs n'ayant pas réagi, et des éventuels sous-produits non désirés.

2. Procédé selon la revendication 1, dans lequel la température réactionnelle est comprise entre 25° C et 120°C, de préférence comprise entre 25°C et 100°C, de préférence encore comprise entre 25°C et 70°C, typiquement ente 30°C et 60°C, par exemple environ 50°C.

3. Procédé selon la revendication 1, dans lequel la pression réactionnelle est comprise entre 4 MPa et 15 MPa, de préférence comprise entre 5 MPa et 12 MPa.

4. Procédé selon la revendication 1, dans lequel le réacteur est choisi parmi les réacteurs munis d'une boucle de recirculation, les réacteurs à turbine auto-aspirante, les réacteurs munis d'un système dit à venturi, les réacteurs à lit rotatif, les réacteurs tubulaires, et autres.

5. Procédé selon la revendication 1, dans lequel l'initiateur radicalaire est choisi parmi l'azo-*bis*-*iso*-butyronitrile, les peroxydes minéraux ou organiques, le sulfate de mercure, le sulfate de palladium, le sulfate de césium, le K₂P₂O₈, le CaO₂, les halogènes, de préférence parmi les peroxydes de bis-(alcane-sulfonyle) de formule CₙH₂ₙ₊₁-SO₂-O-O-SO₂O-CₙH₂ₙ₊₁, où n est tel que défini précédemment, les initiateurs de formule CₙH₂ₙ₊₁-SO₂-O-O-SO₂OX, où n est tel que défini précédemment et X représente l'hydrogène, un métal alcalin ou alcalino-terreux, le zinc ou l'aluminium, l'acide de Caro et ses sels avec un ou plusieurs métaux alcalin ou alcalino-terreux, et l'acide de Marshall et ses sels avec un ou plusieurs métaux alcalin ou alcalino-terreux.

6. Procédé selon la revendication 1, dans lequel l'alcane et le trioxyde de soufre sont ajoutés au fur et à mesure de leur consommation et de la quantité d'acide alcane-sulfonique soutiré, tout en maintenant en maintenant le ratio molaire (alcane de formule CₙH₂ₙ₊₂)/(trioxyde de soufre) compris entre 4/1 et 2/1, de préférence entre 3,5/1 et 2,5/1, typiquement environ égal à 3, et le ratio molaire (acide alcane-sulfonique)/(trioxyde de soufre) entre 70/30 et 95/5.

7. Procédé selon la revendication 1, dans lequel le solvant est l'acide méthane-sulfonique, l'alcane est le méthane et le produit soutiré en continu est l'acide méthane-sulfonique.

8. Milieu réactionnel comprenant un acide alcane-sulfonique de formule CₙH₂ₙ₊₁SO₃H, où n est un entier compris entre 1 et 20 bornes incluses, de préférence entre 1 et 12 bornes incluses, de préférence encore entre 1 et 6 bornes incluses, et par exemple égal à 1, 2 ou 3, du trioxyde de soufre SO₃, et un alcane de formule CₙH₂ₙ₊₂, où n est tel que défini précédemment, milieu réactionnel dans lequel :
- le ratio molaire (acide alcane-sulfonique)/(SO₃) est compris entre 70/30 et 95/5, et
- le ratio molaire (alcane)/(SO₃) est compris entre 4/1 et 2/1, de préférence entre 3,5/1 et 2,5/1, typiquement égal à environ 3.

9. Milieu réactionnel selon la revendication 8, comprenant de l'acide méthane-sulfonique (CH₃SO₃H), du trioxyde de soufre SO₃, et du méthane (CH4) dans lequel :
- le ratio molaire (acide méthane-sulfonique)/(SO₃) est compris entre 70/30 et 95/5, et
- le ratio molaire (méthane)/(SO₃) est compris entre 4/1 et 2/1, de préférence entre 3,5/1 et 2,5/1, typiquement égal à environ 3.

## Patentansprüche

1. Verfahren zur industriellen kontinuierlichen Herstellung von Alkansulfonsäure der Formel CₙH₂ₙ₊₁SO₃H aus dem Alkan der Formel CₙH₂ₙ₊₂, wobei n eine ganze Zahl zwischen 1 und 20 einschließlich der Grenzen, vorzugsweise zwischen 1 und 12 einschließlich der Grenzen, noch stärker bevorzugt zwischen 1 und 6 einschließlich der Grenzen und zum Beispiel gleich 1, 2 oder 3 darstellt, wobei das Verfahren mindestens die folgenden Schritte umfasst:
a) Beschicken eines Reaktors mit Alkansulfonsäure CₙH₂ₙ₊₁SO₃H, wobei n wie vorstehend definiert ist,
b) Erhitzen der Alkansulfonsäure auf eine Temperatur zwischen 25 °C und 120 °C, vorzugsweise zwischen 25 °C und 100 °C, noch stärker bevorzugt zwischen 25 °C und 70 °C,
c) Einbringen einer Menge an Schwefeltrioxid derart, dass das Molverhältnis (Alkansulfonsäure)/(Schwefeltrioxid) zwischen 70/30 und 95/5 beträgt,
d) Einbringen des Alkans unter einem Druck zwischen 4 MPa und 15 MPa, vorzugsweise zwischen 5 MPa und 12 MPa,
d) Einbringen eines Radikalstarters,
e) kontinuierliches Durchführen der Reaktion, wobei man gleichzeitig, nacheinander oder alternativ die drei folgenden Teilschritte e1), e2) und e3) durchführt:
e1) Zugabe von Schwefeltrioxid derart, dass das in Schritt c) definierte anfängliche Molverhältnis (Alkansulfonsäure)/(Schwefeltrioxid) aufrechterhalten wird,
e2) Zugabe des Alkans derart, dass ein Molverhältnis (Alkan)/(Schwefeltrioxid) zwischen 4/1 und 2/1, vorzugsweise zwischen 3,5/1 und 2,5/1, aufrechterhalten wird, üblicherweise derart, dass ein Molverhältnis (Alkan)/(Schwefeltrioxid) gleich etwa 3 aufrechterhalten wird, und
e3) kontinuierliches Entnehmen der Alkansulfonsäure der Formel CₙH₂ₙ₊₁SO₃H und
f) Gewinnung der Alkansulfonsäure der Formel CₙH₂ₙ₊₁SO₃H nach eventueller Abtrennung nicht umgesetzter Reagenzien und jeglicher eventueller unerwünschter Nebenprodukte.

2. Verfahren nach Anspruch 1, wobei die Reaktionstemperatur zwischen 25 °C und 120 °C, vorzugsweise zwischen 25 °C und 100 °C, noch stärker bevorzugt zwischen 25 °C und 70 °C, üblicherweise zwischen 30 °C und 60 °C, zum Beispiel etwa 50 °C beträgt.

3. Verfahren nach Anspruch 1, wobei der Reaktionsdruck zwischen 4 MPa und 15 MPa, vorzugsweise zwischen 5 MPa und 12 MPa, beträgt.

4. Verfahren nach Anspruch 1, wobei der Reaktor aus mit einer Rezirkulationsschleife ausgestatteten Reaktoren, Reaktoren mit einer selbst-ansaugenden Turbine, mit einem Venturi-System ausgestatteten Reaktoren, Drehbettreaktoren, Rohrreaktoren und anderen ausgewählt ist.

5. Verfahren nach Anspruch 1, wobei der Radikalstarter aus Azo-bis-isobutyronitril, anorganischen oder organischen Peroxiden, Quecksilbersulfat, Palladiumsulfat, Cäsiumsulfat, K₂P₂O₃, CaO₂, Halogenen, vorzugsweise aus Bis(alkansulfonyl)peroxiden der Formel CₙH₂ₙ₊₁-SO₂-O-O-SO₂O-CₙH₂ₙ₊₁, wobei n wie vorstehend definiert ist, Startern der Formel CₙH₂ₙ₊₁-SO₂-O-O-SO₂OX, wobei n wie vorstehend definiert ist und X für Wasserstoff, ein Alkali- oder Erdalkalimetall, Zink oder Aluminium steht, Caroscher Säure und deren Salzen mit einem oder mehreren Alkali- oder Erdalkalimetallen sowie Marshallsäure und deren Salzen mit einem oder mehreren Alkali- oder Erdalkalimetallen ausgewählt ist.

6. Verfahren nach Anspruch 1, wobei das Alkan und das Schwefeltrioxid in dem Maße, in dem sie verbraucht werden, und je nach der Menge der entnommenen Alkansulfonsäure zugegeben werden, wobei man das Molverhältnis (Alkan der Formel CₙH₂ₙ₊₂) / (Schwefeltrioxid) zwischen 4/1 und 2/1, vorzugsweise zwischen 3,5/1 und 2,5/1, üblicherweise gleich etwa 3, und das Molverhältnis (Alkansulfonsäure)/(Schwefeltrioxid) zwischen 70/30 und 95/5 hält hält.

7. Verfahren nach Anspruch 1, wobei das Lösungsmittel Methansulfonsäure ist, das Alkan Methan ist und das kontinuierlich entnommene Produkt Methansulfonsäure ist.

8. Reaktionsmedium, umfassend eine Alkansulfonsäure der Formel CₙH₂ₙ₊₁SO₃H, wobei n eine ganze Zahl zwischen 1 und 20 einschließlich der Grenzen, vorzugsweise zwischen 1 und 12 einschließlich der Grenzen, noch stärker bevorzugt zwischen 1 und 6 einschließlich der Grenzen und zum Beispiel gleich 1, 2 oder 3 darstellt, Schwefeltrioxid SO₃ und ein Alkan der Formel CₙH₂ₙ₊₂, wobei n wie vorstehend definiert ist, wobei in dem Reaktionsmedium:
- das Molverhältnis (Alkansulfonsäure)/(SO₃) zwischen 70/30 und 95/5 beträgt und
- das Molverhältnis (Alkan)/(SO₃) zwischen 4/1 und 2/1, vorzugsweise zwischen 3,5/1 und 2,5/1, üblicherweise gleich etwa 3 beträgt.

9. Reaktionsmedium nach Anspruch 8, umfassend Methansulfonsäure (CH₃SO₃H), Schwefeltrioxid SO₃ und Methan (CH₄), wobei:
- das Molverhältnis (Methansulfonsäure)/(SO₃) zwischen 70/30 und 95/5 beträgt und
- das Molverhältnis (Methan)/(SO₃) zwischen 4/1 und 2/1, vorzugsweise zwischen 3,5/1 und 2,5/1, üblicherweise gleich etwa 3 beträgt.

## Claims

1. Process for continuous industrial preparation of alkanesulfonic acid of formula CₙH₂ₙ₊₁SO₃H starting from the alkane of formula CₙH₂ₙ₊₂, where n represents an integer between 1 and 20 inclusive, preferably between 1 and 12 inclusive, more preferably between 1 and 6 inclusive, and for example equal to 1, 2 or 3, said process comprising at least the following steps:
a) charging a reactor with alkanesulfonic acid CₙH₂ₙ₊₁SO₃H, where n is as defined above,
b) heating said alkanesulfonic acid to a temperature of between 25°C and 120°C, preferably of between 25°C and 100°C, more preferably of between 25°C and 70°C,
c) introducing an amount of sulfur trioxide such that the (alkanesulfonic acid)/(sulfur trioxide) molar ratio is between 70/30 and 95/5,
d) introducing the alkane under a pressure of between 4 MPa and 15 MPa, preferably of between 5 MPa and 12 MPa, d) introducing a free-radical initiator,
e) carrying out the reaction in a continuous manner, by simultaneous, sequential or alternating operation of the following three substeps e1), e2) and e3):
e1) adding sulfur trioxide so as to maintain the initial (alkanesulfonic acid)/(sulfur trioxide) molar ratio defined in step c),
e2) adding the alkane so as to maintain an (alkane)/(sulfur trioxide) molar ratio between 4/1 and 2/1, preferably of between 3.5/1 and 2.5/1, typically so as to maintain an (alkane)/(sulfur trioxide) molar ratio equal to about 3, and
e3) continuously withdrawing the alkanesulfonic acid of formula CₙH₂ₙ₊₁SO₃H, and
f) recovering the alkanesulfonic acid of formula CₙH₂ₙ₊₁SO₃H after optional separation of unreacted reactants and of possible undesired by-products.

2. Process according to Claim 1, wherein the reaction temperature is between 25°C and 120°C, preferably between 25°C and 100°C, more preferably between 25°C and 70°C, typically between 30°C and 60°C, for example about 50°C.

3. Process according to Claim 1, wherein the reaction pressure is between 4 MPa and 15 MPa, preferably between 5 MPa and 12 MPa.

4. Process according to Claim 1, wherein the reactor is chosen from reactors equipped with a recirculation loop, reactors having a self-aspirating turbine, reactors equipped with a "venturi" system, rotating bed reactors, tubular reactors, and others.

5. Process according to Claim 1, wherein the free-radical initiator is chosen from azo-bisisobutyronitrile, inorganic or organic peroxides, mercury sulfate, palladium sulfate, caesium sulfate, K₂P₂O₃, CaO₂, halogens, preferably from bis (alkanesulfonyl) peroxides of formula CₙH₂ₙ₊₁-SO₂-O-O-SO₂O-CₙH₂ₙ₊₁, where n is as defined above, initiators of formula CₙH₂ₙ₊₁-SO₂-O-O-SO₂OX, where n is as defined above and X represents hydrogen, an alkali metal or alkaline earth metal, zinc or aluminium, peroxymonosulfuric acid or salts thereof with one or more alkali metals or alkaline earth metals, and peroxydisulfuric acid or salts thereof with one or more alkali metals or alkaline earth metals.

6. Process according to Claim 1, wherein the alkane and the sulfur trioxide are added as fast as they are consumed and as the amount of alkanesulfonic acid is withdrawn, while maintaining the (alkane of formula CₙH₂ₙ₊₂) / (sulfur trioxide) molar ratio between 4/1 and 2/1, preferably between 3.5/1 and 2.5/1, typically equal to about 3, and the (alkanesulfonic acid)/(sulfur trioxide) molar ratio between 70/30 and 95/5.

7. Process according to Claim 1, wherein the solvent is methanesulfonic acid, the alkane is methane and the product continuously withdrawn is methanesulfonic acid.

8. Reaction mixture comprising an alkanesulfonic acid of formula CₙH₂ₙ₊₁SO₃H, where n is an integer between 1 and 20 inclusive, preferably between 1 and 12 inclusive, more preferably between 1 and 6 inclusive, and for example equal to 1, 2 or 3, sulfur trioxide SO₃, and an alkane of formula CₙH₂ₙ₊₂, where n is as defined above, reaction mixture wherein:
- the (alkanesulfonic acid)/(SO₃) molar ratio is between 70/30 and 95/5, and
- the (alkane)/(SO₃) molar ratio is between 4/1 and 2/1, preferably between 3.5/1 and 2.5/1, typically equal to about 3.

9. Reaction mixture according to Claim 8, comprising methanesulfonic acid (CH₃SO₃H), sulfur trioxide SO₃, and methane (CH₄), wherein:
- the (methanesulfonic acid)/(SO₃) molar ratio is between 70/30 and 95/5, and
- the (methane)/(SO₃) molar ratio is between 4/1 and 2/1, preferably between 3.5/1 and 2.5/1, typically equal to about 3.
